# EUROPÄISCHE PATENTSCHRIFT

(11) **EP 0 316 797 B1**
(45) Veröffentlichungstag und Bekanntmachung des Hinweises auf die Patenterteilung: **20.10.1993**
(21) Anmeldenummer: 88118820.5
(22) Anmeldetag: 11.11.1988
(51) Int. Cl.: C12N 15/53, C12N 15/82, A01H 1/00, A01H 5/00

(54) **Pflanzen mit modifizierter Blütenfarbe und gentechnologische Verfahren zur ihrer Herstellung**
Plants with a modified flowering colour and genetic engineering method for their production
Plantes avec des couleurs florales modifiées ainsi qu'une méthode pour leur préparation par génie génétique

(30) Priorität: 13.11.1987 DE 3738657
(43) Veröffentlichungstag der Anmeldung: 24.05.1989
(73) Patentinhaber: Max-Planck-Gesellschaft zur Förderung der Wissenschaften e.V., D-37073 Göttingen (DE)
(72) Erfinder: Meyer, Peter, Dr. Dipl.-Biol., D-5000 Köln 50 (DE); Heidmann, Iris, D-5000 Köln 40 (DE); Saedler, Heinz, Prof. Dr. Dipl.-Biol., D-5000 Köln 30 (DE); Forkmann, Gert, Dr. Dipl.-Biol., D-7400 Tübingen (DE)
(74) Vertreter: VOSSIUS & PARTNER

(56) Entgegenhaltungen:
- NATURE, Band 330, 17. Dezember 1987, Seiten 677-678; P. MEYER et al.: "A new petunia flower colour generated by transformation of a mutant with a maize gene"
- THE EMBO JOURNAL, Band 6, Nr. 2, 1987, Seiten 287-294, IRL Press Ltd, Oxford, GB; Z. SCHWARZ-SOMMER et al.: "Influence of transposable elements on the structure and function of the A1 gene of Zea mays"
- Z. PFLANZENPHYSIOL., Band 63, 1970, Seiten 31-43; D. HESS: "Versuche zur Transformation an höheren Pflanzen: genetische Charakterisierung einiger mutmasslich transformierter Pflanzen"

## Beschreibung

Die Erfindung betrifft Pflanzen mit modifizierter Blütenfarbe, die eine gentechnologisch eingebrachte DNA-Sequenz aufweisen, die für ein Protein mit der enzymatischen Aktivität einer Dihydroflavonol-4-Reduktase mit erweiterter Substratspezifität für Dihydrokaempferol codiert.
Ferner betrifft die Erfindung gentechnologische Verfahren zur Herstellung von Pflanzen mit modifizierter Blütenfarbe und ein bei diesem Verfahren verwendbares Vektorsystem, das gegebenenfalls einen selektierbaren Marker aufweist.

Die Blütenfarbe von Pflanzen wird durch von der Pflanze synthetisierte Farbstoffe verursacht.
Bekanntlich besteht eine große Nachfrage nach Pflanzen mit neuen Blütenfarben. Bisher wurde diese Nachfrage dadurch zu befriedigen versucht, daß man durch Kreuzung Pflanzen mit neuen Blütenfarben züchtete. Die derartige "klassische" Neuzüchtung von Pflanzen war jedoch durch die genetischen Kompatibilitätsgrenzen der einzelnen zur Kreuzung verwendeten Pflanzenarten beschränkt.
Beispielsweise konnten mit diesen Methoden keine lachsroten Petunien (Petunia hybrida) gezüchtet werden, da die genetische Information für das Schlüsselenzym des Syntheseweges des entsprechenden Farbstoffes bei Petunienarten nicht vorkommt.
Aus anderen Pflanzenarten, die über diese genetische Information verfügen, z.B. aus Mais (Zea mays) oder Löwenmäulchen (Antirrhinum majus) kann sie nicht in Petunia hybrida eingekreuzt werden. Petunia hybrida und Zea mays bzw. Antirrhinum majus sind genetisch inkompatibel.
Auch bestand im Stand der Technik die Auffassung, daß selbst wenn es gelingen wurde, Gene aus monokotylen Pflanzen, wie Zea mays, in dikotyle Pflanzen, wie Petunia hybrida, einzubringen, diese nicht mehr aktiv sein würden.
Aufgrund dieser Schwierigkeiten konnte die Nachfrage nach Pflanzen mit neuen Blütenfarben bisher nur im beschränkten Umfang befriedigt werden.

Der Erfindung liegt somit die Aufgabe zugrunde, auf gentechnologischem Wege Pflanzen mit modifizierter Blütenfarbe bereitzustellen und ein gentechnologisches Verfahren zu schaffen, das die Herstellung solcher Pflanzen gestattet.
Eine weitere Aufgabe ist es, einen bei diesem Verfahren verwendbaren Vektor bereitzustellen, der gegebenenfalls einen selektierbaren Marker aufweist.

Diese erfindungsgemäße Aufgabe wird durch die Bereitstellung der in den Patentansprüchen gekennzeichneten Ausführungsformen gelöst.

Die Figuren zeigen:
- Figur 1:: Vektor p35A1
- Figur 2:: links: Aufgrund der in Spuren enthaltenden Cyanidin- und Delphinidin-Derivate weist die Mutante RL01 eine blaßrosa Blütenfarbe auf.
rechts: Die Transformante RP235-15 trägt das A1-Gen aus Zea mays, in Form einer cDNA. Dieses Gen befähigt sie, Pelargonidin-3-Glycosid zu synthetisieren. Daher weist sie eine lachsrote Blütenfarbe auf.
- Figur 3:: Transkription des A1-Gens aus Zea mays in transgenen Petunia hybrida-Pflanzen. Die Transformante RP235-15 weist einen starken mRNA-Gehalt des A1-Gens aus Zea mays auf (Bahn 1). In der Transformante RP235-12, die eine unveränderte Blütenfarbe hat, liegen keine Transkripte des A1-Gens vor (Bahn 2). Auch die nicht transformierte Mutante RL01 weist keine Transkripte des A1-Gens auf (Bahn 3) (Kontrolle).
- Figur 4:: Schematische Darstellung eines Ausschnittes der Anthocyan-Biosynthese.

Erfindungsgemäß werden die Pflanzen mit modifizierter Blütenfarbe dadurch konstruiert, daß man gentechnologisch in die Pflanze eine DNA-Sequenz einbringt, die für ein Protein mit der enzymatischen Aktivität einer Dihydroflavonol-4-Reduktase mit erweiterter Substratspezifität für Dihydrokaempferol codiert (nachfolgend kurz als DFR, in der Literatur oft auch als DQR bezeichnet).
Der Ausdruck "Protein mit der enzymatischen Aktivität einer Dihydroflavonol-4-Reduktase mit erweiterter Substratspezifität für Dihydrokaempferol" bezeichnet ein Protein, das einem in der Natur vorkommenden Enzym mit der genannten Spezifität entspricht, aber auch Proteine, die nicht natürlich vorkommen und die genannte Spezifität aufweisen. Beispiele sind Fusionsproteine oder Proteine, die nur einen enzymatisch aktiven Teilbereich des natürlich vorkommenden Enzyms enthalten.

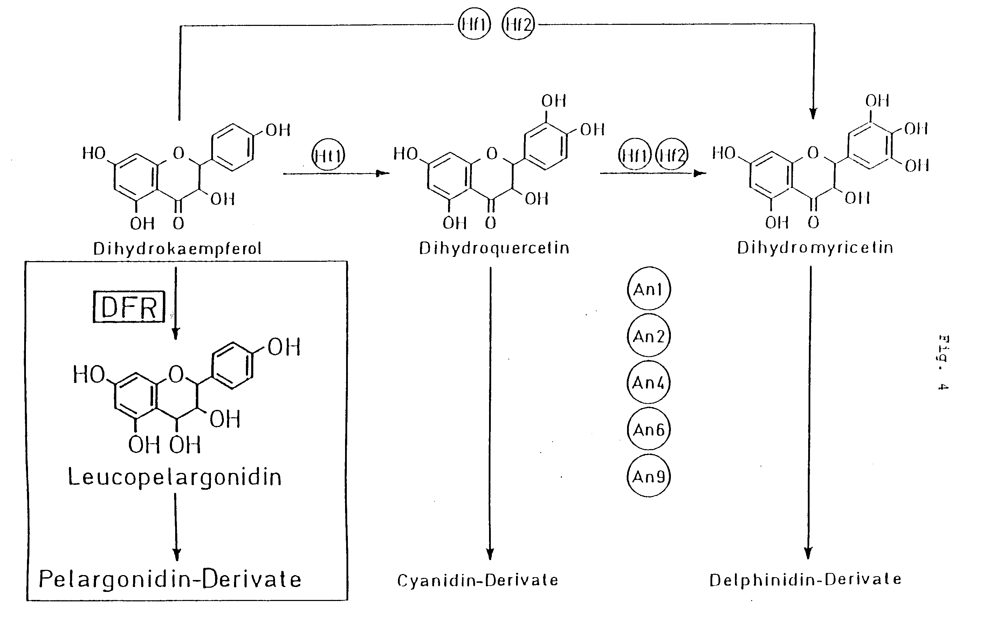

Wenn die neu eingebrachte genetische Information in den Zellen der Pflanze unter der Kontrolle eines geeigneten Promotors steht, dann wird sie exprimiert. Dabei wird das Enzym DFR in den Zellen synthetisiert.
Sofern in den Zellen als Zwischenprodukt der Anthocyanbiosynthese Dihydrokaempferol anfällt, wird dies vom synthetisierten Enzym DFR zu Leucopelargonidin reduziert; vgl. Fig. 4. Die ohnehin in den Zellen vorliegenden weiteren Enzyme modifizieren das Leucopelargonidin zu lachsroten Anthocyan-Farbstoff-Pelargonidin-3-Glycosiden.

Spenderpflanzen, aus denen das DFR-Gen isoliert werden kann, sind dem Fachmann bekannt. Beispiele sind Pelargonidin-produzierende Pflanzen, wie Zea mays, Antirrhinum majus, Matthiola incana (Levkoje) oder Callistephus chinensis (Aster).
Außerdem sind dem Fachmann Akzeptorpflanzen bekannt, bei denen zwar Dihydrokaempferol im Anthocyanbiosyntheseweg anfällt, jedoch mangels einer Dihydroflavonol-4-Reduktase mit Substratspezifität für Dihydrokaempferol nicht zu Leucopelargonidin umgesetzt werden kann. Beispiele sind Petunia hybrida oder Nicotiana alata (Ziertabak).

In einer bevorzugten Ausführungsform der vorliegenden Erfindung werden als DFR-DNA-Spenderpflanze die Pelargonidin-produzierenden Pflanzen Zea mays (monokotyl) oder Antirrhinum majus und als DQR-DNA-Akzeptorpflanze Petunia hybrida (dikotyl) verwendet.
In Zea mays wird das Enzym DFR vom A1-Gen codiert. Im Aleuron von Zea mays wird von diesem Enzym Dihydroquercetin in Leucocyanidin umgesetzt und Dihydrokaempferol zu Leucopelargonidin, wodurch die Bildung von Cyanidin-Derivaten und von Pelargonidin-Derivaten eingeleitet wird. Diese Dihydroflavonol-4-Reduktase aus Zea mays weist eine erweiterte Substratspezifität für Dihydrokaempferol auf.

Erfindungsgemäß lassen sich gentechnologisch mit der DFR-DNA aus Zea mays Varianten von Petunia hybrida konstruieren, die eine lachsrote Blütenfarbe aufweisen, weil sie mit Hilfe der gentechnologisch eingebrachten DFR-DNA aus Zea mays den lachsroten Anthocyan-Farbstoff Pelargonidin-3-Glycosid synthetisieren können.

Derartige Varianten von Petunia hybrida waren bisher nicht bekannt. Dies beruhte darauf, daß die in Petunien üblicherweise vorkommende Dihydroflavonol-4-Reduktase zwar mit verhältnismäßig hoher Effizienz Dihydromyricetin und mit niedrigerer Effizienz Dihydroquercetin umsetzt, jedoch kein Dihydrokaempferol umsetzen kann; vgl. Fig. 4. Diesem natürlich in Petunia hybrida vorkommenden Enzym fehlt also die Substratspezifität für Dihydrokaempferol. Deshalb sind die in Petunien natürlich vorkommenden Anthocyan-Farbstoffe lediglich Delphinidin-Derivate (dunkelblau) bzw. Cyanidin-Derivate (blaurot); vgl. Fig. 4. Zu diesen Derivaten gehören beispielsweise Glycoside.

In einer erfindungsgemäß bevorzugten Ausfuhrungsform wird als Akzeptorpflanze eine Mutante von Petunia hybrida verwendet, die eine genetische Defizienz für die Aktivität der 3′- und 3′,5′-Hydroxylasen aufweist; vgl. die in Fig. 4 als Ht1 bzw. als Hf1 und Hf2 bezeichneten Enzyme.
Aufgrund dieser Defizienz werden bei dieser Pflanze Dihydrokaempferol und Kaempferol akkumuliert, da diese Verbindungen nicht in der Anthocyanbiosynthese zu Dihydroquercetin und Dihydromyricetin umgesetzt werden; vgl. Fig. 4. Somit fehlen der Pflanze die ausgehend von diesen Zwischenprodukten synthetisierten Anthocyan-Farbstoffe, nämlich Cyanidin-Derivate (blaurot) und Delphinidin-Derivate 3 (dunkelblau). Ihre Blütenfarbe ist deshalb im wesentlichen weiß bzw. blaßrosa.

Die Herstellung derartiger Defektmutanten ist dem Fachmann bekannt. Ein Beispiel, die Mutante RL01, wird von G. Stotz in Theor. Appl. Genet. 70 (1985), S. 300-305, beschrieben. Diese Mutante aus der Petunia hybrida-Sammlung in Tübingen ist ein Derivat der Linie R4, die früher für genetische und enzymatische Untersuchungen der B-Ringhydroxylierung und der Flavonolbildung verwendet wurde. Da die Mutante RL01 rezessive Allele an den Ht1- und Hf1-Loci enthält, weist sie keine Flavonoid-3′-Hydroxylaseaktivität und nur eine geringe Flavonoid-3′,5′-Hydroxylaseaktivität auf. Aufgrunddessen werden in dieser Pflanze Cyanidin-Derivate und Delphinidin-Derivate nur in geringen Mengen hergestellt. Daher hat sie eine blaßrosa Blütenfarbe, Anthocyan-Farbstoffe des Pelargonidin-Typs werden von dieser Mutante nicht synthetisiert. Wie bereits vorstehend erläutert, wird Dihydrokaempferol aufgrund der oben erläuterten genetischen Defekte und aufgrund der Unfähigkeit, Dihydrokaempferol zu Leucopelargonidin umzusetzen, akkumuliert; vgl. Fig. 4. Wenn der Mutante RL01 das Anthocyan-Synthese-Zwischenprodukt Leucopelargonidin in üblicher Weise angeboten wird, wird dies zu Pelargonidin-3-Glycosid umgesetzt und die Blütenbehälter färben sich lachsrot; vgl. Fig. 4. Daraus folgt, daß alle anderen Gene der Anthocyan-Synthese durch dominante Allele repräsentiert werden. Die Mutante RL01 weist somit lediglich einen Defekt in den für die Hydroxylierung von Dihydrokaempferol verantwortlichen Enzymen auf.

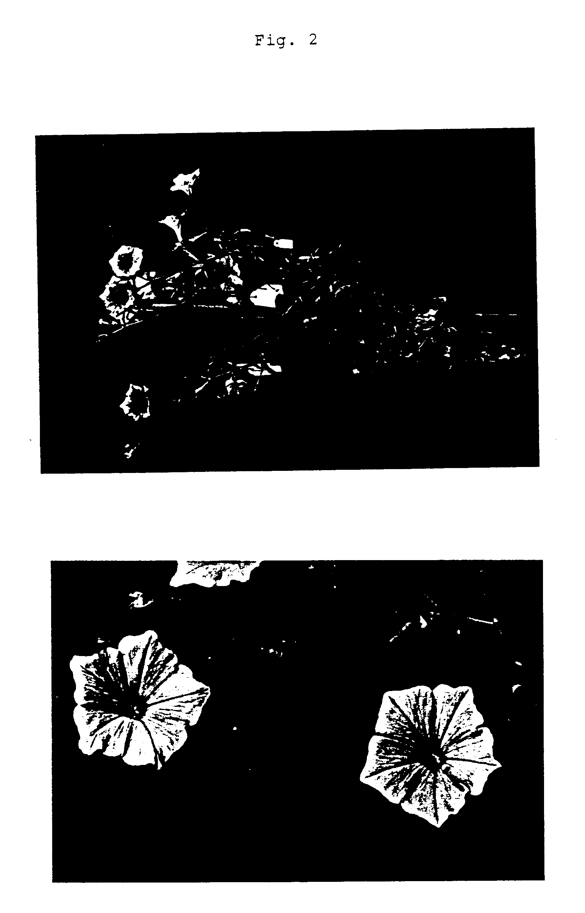

Wenn in die vorstehend erläuterten Mutanten von Petunia hybrida, beispielsweise in die Mutante RL01, erfindungsgemäß das DFR-Gen eingebracht wird, dann wird die Blütenfarbe zu einem Lachsrot modifiziert, das häufig die Petunia hybridatypischen Blütenzeichnungen aufweist; vgl. Fig. 2.

Die gentechnologische Einführung der die DFR codierenden DNA-Sequenz läßt sich nach üblichen Techniken bewerkstelligen, beispielsweise durch Mikroinjektion (A. de la Pena, H. Lörz, & J. Schell, Nature 325 (1987), Seiten 274-276), Elektroporation (M. E. Fromm, L. P. Taylor & V. Walbot, Nature 319 (1986), Seiten 791-793), Transformation (F. A. Krens, L. Molendijk, G. J. Wullems, R.A. Schilperoort, Nature 296 (1982), Seiten 72-74), Transfer von entsprechenden Ti-Plasmiden (L. Marton, G.J. Wullems, L. Molendijk, R.A. Schilperoort, Nature 277 (1979), Seiten 129-131) und durch Liposomen-vermittelten Transfer (R. T. Fraley, Plant Mol. Biology 2, Seiten 5 ff.).
Für das Einbringen der DNA sind übliche Vektoren, beispielsweise das Ti-Plasmid, geeignet.
Derartige Vektorsysteme sind erläutert in L. Herrera Estrella et al., Nature 303 (1983), 209; L. Herrera Estrella et al., EMBO J. 2 (1983), 987; J. P. Hernalsteens et al., Nature 287 (1980), 654 und in R. T. Fraley et al., Proc. Natl. Acad. Sci. USA 80 (1983), 4803.
Außerdem wird das Ti-Plasmid-System beispielsweise in EP-A1-0 116 718 beschrieben.

Vorzugsweise wird die DNA-Sequenz in Protoplasten der Akzeptorpflanze eingebracht, die in M-Phase synchronisiert wurden. Diese Transformationstechnik wird von P. Meyer et al. in Mol. Gen. Genet. 201 (1985), S. 513-528, beschrieben.

Je nach verwendetem Vektorsystem läßt sich die DNA-Sequenz neben Protoplasten auch in Zellen und Gewebe der Pflanze einbringen.

Vorzugsweise wird der rekombinante Vektor p35A1 verwendet. Unter der Kontrolle des in diesem Vektor enthaltenden 35S-Promotors aus CaMV (Cauliflower Mosaic Virus) wird das in Form der cDNA clonierte A1-Gen aus Zea mays konstitutiv exprimiert. Ferner wird mit diesem Vektor neben dem DFR-Gen gleichzeitig ein Kanamycin-Resistenz-Gen auf die Pflanze übertragen, so daß mit dem Vektor transformierte Pflanzen leicht aufgrund ihrer Kanamycin-Resistenz selektiert werden können.

Die Beispiele erläutern die Erfindung:
Ergänzende Erläuterungen der DNA-Rekombinations-Techniken finden sich in Maniatis et al., "Molecular Cloning", CSH Laboratory, Cold Spring Harbor, New York (1982).

### Beispiel 1:

### Konstruktion des Vektors p35A1

Die Isolierung einer cDNA-Sequenz des A1-Gens und insbesondere die des Typ 2-A1-Gens aus Zea mays wird von Z. Schwarz-Sommer et al. in EMBO J. 2 (1987), S. 287-294, beschrieben.

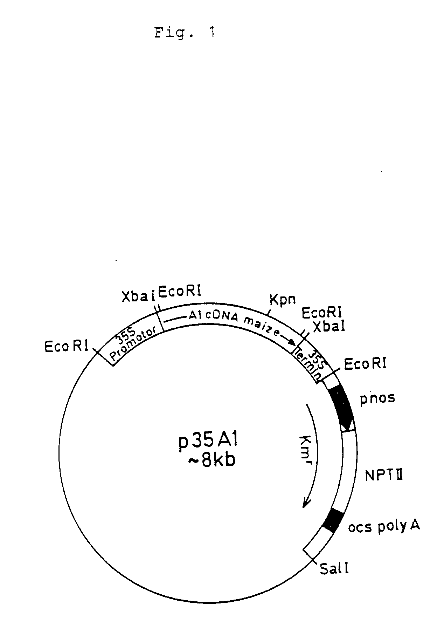

Zunachst werden in einem 1320 bp EcoRI-Fragment des vollständigen cDNA-Clons eines Typ 2-A1-Gens von Zea mays die EcoRI-Restriktionsspaltstellen aufgefüllt und XbaI-Linker wurden angehängt, die die EcoRI-Restriktionsspaltstellen rekonstituieren.
Das erhaltene XbaI-Fragment wird in die einzige XbaI-Restriktionsspaltstelle des Plasmids pCKan1 eingesetzt. Damit befindet es sich in diesem Plasmid zwischen dem 35S-Promotor und der Terminations-Sequenz von CaMV (Cauliflower Mosaic Virus). Das große EcoRI-Fragment des Plasmids pCKan1 wurde ursprünglich vom Plasmid pLGV11 abgeleitet, das ein Kanamycin-Resistenz-Gen trägt. Deshalb konnen die mit dem Plasmid pCKan1 transformierten Pflanzenzellen aufgrund ihrer Kanamycin-Resistenz selektiert werden. Das Plasmid pLGV11 entspricht dem Plasmid pLGV1103, in dem das SalI-Fragment Tn903 deletiert wurde (R. Hain et al., Plant Cell Rep. 2 (1983), S. 244-247).
Das bei dieser Konstruktion erhaltene Plasmid ist das Plasmid p35A1; vgl. Fig. 1. Es wurde bei der Deutschen Sammlung von Mikroorganismen am 14. Oktober 1987 unter der Hinterlegungsnummer DSM 4275 hinterlegt.

### Beispiel 2

### Konstruktion einer Petunia hybrida mit lachsroter Blütenfarbe

In M-Phase synchronisierte Protoplasten der Petunia hybrida-Mutante RL01 werden mit dem Plasmid p35A1 aus Beispiel 1 nach Meyer et al., a.a.O., transformiert.

Nach der Transformation werden Mikrocalli herangezüchtet und in einer Bead-Type-Kultur in V47-Medium mit auf 100 mosm pro Woche reduzierter Osmolarität selektiert (R.D. Shilito et al., Plant Cell Rep. 2 (1983), S. 244-247; H. Binding, Z.Pflanzenphysiol. 74 (1974), S. 327-356). Kanamycin-resistente Mikrocalli werden in Regenerierungs-Medium überführt, sobald sie einen Durchmesser von 3-5 mm erreicht haben. Sie werden drei Wochen auf Re27/6-Medium (MS-Medium) mit 2 mg/l BAP (Benzylaminopurin) und 2 mg/l IAA (Indolessigsaure) (T. Murashige, Physiol. Plant. 15 (1962) S. 473-497) und dann auf Re17/3-Medium transferiert (MS-Medium mit 1 mg/l BAP und 1 mg/l IAA). Die Sprosse werden auf MS-Medium ohne Hormone bewurzelt. Alle Medien enthalten 50 mg/l Kanamycin.

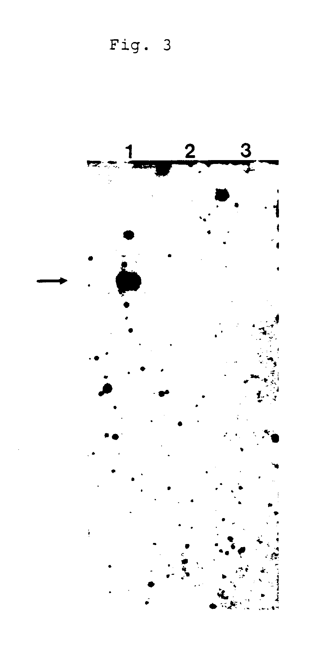

Von den überlebenden Calli exprimieren 2 % das vom Plasmid p35A1 codierte Kanamycin-Resistenz-Gen und sind deshalb Kanamycin-resistent. Aus jedem transformierten Callus werden zwei Pflanzen regeneriert.
Von den ersten 15 blühenden Transformanten zeigen zwei eine lachsrote Blütenfarbe bei beiden regenerierten Pflanzen; vgl. Fig. 2, rechts. Das Auftreten des für diese lachsrote Blütenfarbe verantwortlichen Pelargonidin-Farbstoffes korreliert mit der Transkription der A1-cDNA aus Zea mays. Diese wurde mit dem Plasmid p35A1, in dem sie unter der Kontrolle des 35S-Promotors des CaMV steht, übertragen. Die Transkription wird durch mRNA-Extraktion aus den Blättern nach J. Logemann gezeigt (Analytical Biochemistry 163 (1987), S. 16-20). Dabei wird Hybond^{R}-mAP (Amersham) nach den Anweisungen des Herstellers verwendet. Durch Hybridisierung mit einem EcoRI-XbaI-Restriktionsfragment der A1-cDNA als Sondenmolekül wird die aus den Blättern isolierte A1-mRNA in einem Northern Blot sichtbar gemacht (A.P. Feinberg et al., Anal. Biochem. 132 (1983), S. 6-13 und 137 (1983), 266-267); vgl. Fig. 3.

Die Transformante RP235-15, die eine einheitliche lachsrote Färbung der Blüte aufweist und bei der die Transkription des A1-Gens eindeutig nachgewiesen werden konnte, wird zur Flavonoid-Analyse verwendet. Dabei wird sie mit der Mutante RL01 verglichen. Die Analyse erfolgt nach Standardmethoden J.B. Harborne "Comparative Biochemistry of the Flavonoids", Academic Press, London and New York (1967).
Sowohl in der Mutante RL01 als auch in der Transformante RP235-15 sind geringe Mengen Cyanidin-3-Glycosid, Cyanidin-3-Glycosylglycosid und Delphinidin-3-Glycosid enthalten. Jedoch sind nur in der Transformante RP235-15 darüber hinaus Pelargonidin-3-Glycosid und Pelargonidin-3-Glycosylglycosid in großen Mengen enthalten. Bei der Transformante RP235-15 ist das spektroskopische Anthocyan-Maximum gegenüber der Mutante RL01 von 528 nm auf 512 nm verschoben. Außerdem sind in der Transformante RP235-15 Dihydrokaempferol gar nicht und Kaempferol nur in Spuren nachweisbar. Wie bereits vorstehend erläutert, werden diese Substanzen in der Mutante RL01 akkumuliert.

Aus dieser Analyse ergibt sich, daß mit Hilfe des Expressionsprodukts des in die Transformante RP235-15 eingebrachten A1-Gens aus Zea mays ein neuer Biosyntheseweg in Petunia hybrida geschaffen wurde. Bemerkenswerterweise wurde dieser neue Biosyntheseweg in der dikotylen Pflanze Petunia hybrida mit Hilfe der cDNA eines Gens aus der monokotylen Pflanze Zea mays geschaffen.
Außerdem stellt das mit dem Vektor p35A1 eingebrachte A1-Gen einen leicht erkennbaren Marker der Mutante RL01 dar. Wenn dieser Marker vorhanden ist, dann verändert sich die Blütenfarbe von blaßrosa nach lachsrot. Somit ist phänotypisch mit freiem Auge erkennbar, ob die Transformation mit dem Vektor p35A1 erfolgreich war.

## Patentansprüche (Patentansprüche für folgende(n) Vertragsstaat(en): BE, DE, FR, GB, IT, NL)

1. Natürlich nicht zur Reduktion von Dihydrokaempferol befähigte Planze und Pflanzenteile, gekennzeichnet durch eine natürlicherweise in dieser Pflanze nicht vorkommende DNA-Sequenz, die für ein Protein mit der enzymatischen Aktivität einer Dihydroflavonol-4-Reduktase (DFR) mit erweiterter Substratspezifität für Dihydrokaempferol codiert.

2. Pflanze nach Anspruch 1, dadurch gekennzeichnet, daß sie zur Gattung Petunia hybrida gehört.

3. Petunia hybrida nach Anspruch 2, dadurch gekennzeichnet, daß sie einen genetischen Defekt aufweist, aufgrund dessen die zur Erzeugung von Dihydroquercetin und Dihydromyricetin erforderlichen 3'- und 3',5'-Hydroxylasen im wesentlichen fehlen.

4. Verfahren zur Herstellung von Pflanzen mit modifizierter Blütenfarbe, dadurch gekennzeichnet, daß man in eine natürlich nicht zur Reduktion von Dihydrokaempferol befähigte Pflanze gentechnologisch eine DNA-Sequenz einbringt, die für ein Protein mit der enzymatischen Aktivität einer Dihydroflavonol-4-Reduktase mit erweiterter Substratspezifität für Dihydrokaempferol codiert.

5. Verfahren nach Anspruch 4, dadurch gekennzeichnet, daß die DNA-Sequenz aus einer Pelargonidin-produzierenden Pflanze, wie Zea mays oder Antirrhinum majus, stammt.

6. Verfahren nach Anspruch 4 oder 5, dadurch gekennzeichnet, daß die DNA-Sequenz in einen Vektor inseriert ist.

7. Verfahren nach Anspruch 6, dadurch gekennzeichnet, daß die in den Vektor inserierte DNA-Sequenz unter der Kontrolle eines in der Pflanze aktiven Promotors steht.

8. Verfahren nach Anspruch 6 oder 7, dadurch gekennzeichnet, daß der Vektor einen selektierbaren Marker, wie ein Kanamycin-Resistenz-Gen, aufweist.

9. Verfahren nach Anspruch 6 bis 8, dadurch gekennzeichnet, daß der rekombinante Vektor p35A1 (DSM 4275) ist.

10. Verfahren nach einem der Ansprüche 4 bis 9, dadurch gekennzeichnet, daß man die DNA-Sequenz durch Mikroinjektion, Elektroporation, Transformation oder mit dem Ti-Plasmid-System in Protoplasten, Zellen oder Gewebe der Pflanze einbringt.

11. Verfahren nach einem der Ansprüche 4 bis 9, dadurch gekennzeichnet, daß man die DNA-Sequenz in Protoplasten der Pflanze einbringt, die in M-Phase synchronisiert worden sind.

12. Rekombinanter Vektor p35A1 (DSM 4275).

## Patentansprüche (Patentansprüche für folgende(n) Vertragsstaat(en): ES)

1. Natürlich nicht zur Reduktion von Dihydrokaempferol befähigte Planze und Pflanzenteile, gekennzeichnet durch eine natürlicherweise in dieser Pflanze nicht vorkommende DNA-Sequenz, die für ein Protein mit der enzymatischen Aktivität einer Dihydroflavonol-4-Reduktase (DFR) mit erweiterter Substratspezifität für Dihydrokaempferol codiert.

2. Pflanze nach Anspruch 1, dadurch gekennzeichnet, daß sie zur Gattung Petunia hybrida gehört.

3. Petunia hybrida nach Anspruch 2, dadurch gekennzeichnet, daß sie einen genetischen Defekt aufweist, aufgrund dessen die zur Erzeugung von Dihydroquercetin und Dihydromyricetin erforderlichen 3'- und 3',5'-Hydroxylasen im wesentlichen fehlen.

4. Verfahren zur Herstellung von Pflanzen mit modifizierter Blütenfarbe, dadurch gekennzeichnet, daß man in eine natürlich nicht zur Reduktion von Dihydrokaempferol befähigte Pflanze gentechnologisch eine DNA-Sequenz einbringt, die für ein Protein mit der enzymatischen Aktivität einer Dihydroflavonol-4-Reduktase mit erweiterter Substratspezifitat für Dihydrokaempferol codiert.

5. Verfahren nach Anspruch 4, dadurch gekennzeichnet, daß die DNA-Sequenz aus einer Pelargonidin-produzierenden Pflanze, wie Zea mays oder Antirrhinum majus, stammt.

6. Verfahren nach Anspruch 4 oder 5, dadurch gekennzeichnet, daß die DNA-Sequenz in einen Vektor inseriert ist.

7. Verfahren nach Anspruch 6, dadurch gekennzeichnet, daß die in den Vektor inserierte DNA-Sequenz unter der Kontrolle eines in der Pflanze aktiven Promotors steht.

8. Verfahren nach Anspruch 6 oder 7, dadurch gekennzeichnet, daß der Vektor einen selektierbaren Marker, wie ein Kanamycin-Resistenz-Gen, aufweist.

9. Verfahren nach Anspruch 6 bis 8, dadurch gekennzeichnet, daß der rekombinante Vektor p35A1 (DSM 4275) ist.

10. Verfahren nach einem der Ansprüche 4 bis 9, dadurch gekennzeichnet, daß man die DNA-Sequenz durch Mikroinjektion, Elektroporation, Transformation oder mit dem Ti-Plasmid-System in Protoplasten, Zellen oder Gewebe der Pflanze einbringt.

11. Verfahren nach einem der Ansprüche 4 bis 9, dadurch gekennzeichnet, daß man die DNA-Sequenz in Protoplasten der Pflanze einbringt, die in M-Phase synchronisiert worden sind.

12. "Verfahren zur Herstellung des rekombinanten Vektors p35A1 (DSM 4275), dadurch gekennzeichnet, daß man ein 1320 bp EcoRI-Fragment eines vollständigen cDNA-Clons eines Typ 2-A1-Gens aus Zea mays zwischen den 35S-Promotor und die CaMV-Terminations-Sequenz eines Vektors einsetzt, wobei der Vektor zusätzlich ein das Kanamycin-Resistenz-Gen umfassendes Fragment enthält, das vom Plasmid pLGV11 abgeleitet ist."

## Claims (Claims for the following Contracting State(s): BE, DE, FR, GB, IT, NL)

1. Plant and plant parts which are not normally capable of reducing dihydrokaempferol, characterized by a DNA sequence which does not occur naturally in this plant, this sequence coding for a protein having the enzymatic activity of a dihydroflavonol-4 reductase (DFR) with extended substrate specificity for dihydrokaempferol.

2. Plant according to claim 1, characterized in that it belongs to the genus Petunia hybrida.

3. Petunia hybrida according to claim 2, characterized in that it has a genetic defect, as a result of which the 3'- and 3',5'-hydroxylases necessary to produce dihydroquercetin and dihydromyricetin are essentially missing.

4. Process for the production of plants with a modified flower colour, characterized in that a DNA sequence which codes for a protein having the enzymatic activity of a dihydroflavonol-4 reductase with extended substrate specificity for dihydrokaempferol is introduced by gene technology into a plant which is not normally capable of reducing dihydrokaempferol.

5. Process according to claim 4, characterized in that the DNA sequence stems from a pelargonidin-producing plant, such as Zea mays or Antirrhinum majus.

6. Process according to claim 4 or 5, characterized in that the DNA sequence is inserted into a vector.

7. Process according to claim 6, characterized in that the DNA sequence inserted into the vector is under the control of a promoter which is active in the plant.

8. Process according to claim 6 or 7, characterized in that the vector has a selectable marker, such as a kanamycin resistance gene.

9. Process according to claims 6 to 8, characterized in that the recombinant vector is p35A1 (DSM 4275).

10. Process according to one of claims 4 to 9, characterized in that the DNA sequence is introduced into protoplasts, cells or tissue of said plant by micro-injection, electroporation, transformation or with the Ti-plasmid system.

11. Process according to one of claims 4 to 9, characterized in that the DNA sequence is introduced into protoplasts of said plant which have been synchronized in M-phase.

12. Recombinant vector p35A1 (DSM 4275).

## Claims (Claims for the following Contracting State(s): ES)

1. Plant and plant parts which are not normally capable of reducing dihydrokaempferol, characterized by a DNA sequence which does not occur naturally in this plant, this sequence coding for a protein having the enzymatic activity of a dihydroflavonol-4 reductase (DFR) with extended substrate specificity for dihydrokaempferol.

2. Plant according to claim 1, characterized in that it belongs to the genus Petunia hybrida.

3. Petunia hybrida according to claim 2, characterized in that it has a genetic defect, as a result of which the 3'- and 3',5'-hydroxylases necessary to produce dihydroquercetin and dihydromyricetin are essentially missing.

4. Process for the production of plants with a modified flower colour, characterized in that a DNA sequence which codes for a protein having the enzymatic activity of a dihydroflavonol-4 reductase with extended substrate specificity for dihydrokaempferol is introduced by gene technology into a plant which is not normally capable of reducing dihydrokaempferol.

5. Process according to claim 4, characterized in that the DNA sequence stems from a pelargonidin-producing plant, such as Zea mays or Antirrhinum majus.

6. Process according to claim 4 or 5, characterized in that the DNA sequence is inserted into a vector.

7. Process according to claim 6, characterized in that the DNA sequence inserted into the vector is under the control of a promoter which is active in the plant.

8. Process according to claim 6 or 7, characterized in that the vector has a selectable marker, such as a kanamycin resistance gene.

9. Process according to claims 6 to 8, characterized in that the recombinant vector is p35A1 (DSM 4275).

10. Process according to one of claims 4 to 9, characterized in that the DNA sequence is introduced into protoplasts, cells or tissue of said plant by micro-injection, electroporation, transformation or with the Ti-plasmid system.

11. Process according to one of claims 4 to 9, characterized in that the DNA sequence is introduced into protoplasts of said plant which have been synchronized in M-phase.

12. Process for the production of the recombinant vector p35A1 (DSM 4275), characterized in that a 1320 bp EcoRI fragment of a complete cDNA clone of a type 2-A1-gene from Zea mays is inserted between the 35S promoter and the CaMV-termination sequence of a vector, whereby the vector additionally contains a fragment including the kanamycin resistance gene, the fragment being derived from the plasmid pLGV11.

## Revendications (Revendications pour l'(les) Etat(s) contractant(s) suivant(s): BE, DE, FR, GB, IT, NL)

1. Plantes et parties de plantes naturellement incapables d'effectuer une réduction du dihydrocamphérol, caractérisées en ce que ces plantes ne comportent normalement pas une séquence d'ADN qui code pour une protéine ayant l'activité enzymatique d'une dihydroflavonol-4-réductase (DFR) ayant une spécificité élargie de substrat pour le dihydrocamphérol.

2. Plante selon la revendication 1, caractérisée en ce qu'elle appartient au genre Petunia hybrida.

3. Petunia hybrida selon la revendication 2, caractérisée en ce qu'elle présente un défaut génétique en raison duquel les 3'- et les 3',5'-hydroxylases, nécessaires pour la production de la dihydroquercetine et de la dihydromyricétine, en sont essentiellement absentes.

4. Procédé pour préparer des plantes ayant une couleur florale modifiée, procédé caractérisé en ce qu'on introduit par technologie de génie génétique, dans une plante naturellement incapable d'effectuer la réduction du dihydrocamphérol, une séquence d'ADN qui code pour une protéine ayant l'activité enzymatique d'une dihydroflavonol-4-réductase et ayant une spécificité élargie de substrat pour le dihydrocamphérol.

5. Procédé selon la revendication 4, caractérisé en ce que la séquence d'ADN provient d'une plante productrice de pélargonidine, comme Zea mays ou Antirrhinum majus.

6. Procédé selon la revendication 4 ou 5, caractérisé en ce que la séquence d'ADN est insérée dans un vecteur.

7. Procédé selon la revendication 6, caractérisé en ce que la séquence d'ADN insérée dans le vecteur est sous le "contrôle" de commande par un promoteur actif présent dans la plante.

8. Procédé selon la revendication 6 ou 7, caractérisé en ce que le vecteur présente un marqueur permettant une sélection, tel qu'un gêne de résistance à la kanamycine.

9. Procédé selon l'une des revendications 6 à 8, caractérisé en ce que le vecteur recombinant est p35A1 (DSM 4275).

10. Procédé selon l'une des revendications 4 à 9, caractérisé en ce qu'on introduit la séquence d'ADN par microinjection, électroporation, transformation ou à l'aide du système de plasmide de Ti dans des protoplastes, des cellules ou des tissus de la plante.

11. Procédé selon l'une des revendications 4 à 9, caractérisé en ce qu'on introduit la séquence d'ADN dans des protoplastes de la plante, qui ont été synchronisés en phase M.

12. Vecteur recombinant p35A1 (DSM 4275).

## Revendications (Revendications pour l'(les) Etat(s) contractant(s) suivant(s): ES)

1. Plantes et parties de plantes naturellement incapables d'effectuer une réduction du dihydrocamphérol, caractérisées en ce que ces plantes ne comportent normalement pas une séquence d'ADN qui code pour une protéine ayant l'activité enzymatique d'une dihydroflavonol-4-réductase (DFR) ayant une spécificité élargie de substrat pour le dihydrocamphérol.

2. Plante selon la revendication 1, caractérisée en ce qu'elle appartient au genre Petunia hybrida.

3. Petunia hybrida selon la revendication 2, caractérisée en ce qu'elle présente un défaut génétique en raison duquel les 3'- et les 3',5'-hydroxylases, nécessaires pour la production de la dihydroquercetine et de la dihydromyricétine, en sont essentiellement absentes.

4. Procédé pour préparer des plantes ayant une couleur florale modifiée, procédé caractérisé en ce qu'on introduit par technologie de génie génétique, dans une plante naturellement incapable d'effectuer la réduction du dihydrocamphérol, une séquence d'ADN qui code pour une protéine ayant l'activité enzymatique d'une dihydroflavonol-4-réductase et ayant une spécificité élargie de substrat pour le dihydrocamphérol.

5. Procédé selon la revendication 4, caractérisé en ce que la séquence d'ADN provient d'une plante productrice de pélargonidine, comme Zea mays ou Antirrhinum majus.

6. Procédé selon la revendication 4 ou 5, caractérisé en ce que la séquence d'ADN est insérée dans un vecteur.

7. Procédé selon la revendication 6, caractérisé en ce que la séquence d'ADN insérée dans le vecteur est sous le "contrôle" de commande par un promoteur actif présent dans la plante.

8. Procédé selon la revendication 6 ou 7, caractérisé en ce que le vecteur présente un marqueur permettant une sélection, tel qu'un gêne de résistance à la kanamycine.

9. Procédé selon l'une des revendications 6 à 8, caractérisé en ce que le vecteur recombinant est p35A1 (DSM 4275).

10. Procédé selon l'une des revendications 4 à 9, caractérisé en ce qu'on introduit la séquence d'ADN par microinjection, électroporation, transformation ou à l'aide du système de plasmide de Ti dans des protoplastes, des cellules ou des tissus de la plante.

11. Procédé selon l'une des revendications 4 à 9, caractérisé en ce qu'on introduit la séquence d'ADN dans des protoplastes de la plante, qui ont été synchronisés en phase M.

12. Procédé pour préparer le vecteur recombinant p35A1 (DSM 4275), caractérisé en ce qu'on utilise un fragment de 1320 paires de bases, obtenues à l'aide de EcoRI- d'un clône d'ADNc complet d'un type de gêne de type 2 A1 de Zea Mays entre le promoteur 35S et la séquence de terminaison CAMV d'un vecteur, le vecteur contenant en outre un fragment comportant le gêne de résistance à la kanamycine, qui provient du plasmide pLGV11.
